# EUROPEAN PATENT APPLICATION

(11) **EP 4 223 527 A1**
(43) Date of publication of application: **09.08.2023**
(21) Application number: 21875209.5
(22) Date of filing: 15.09.2021
(51) Int. Cl.: B33Y 10/00, B33Y 80/00, B29C 64/10, A61M 16/00, A61M 16/01, A61M 16/16

(54) **ARTIFICIAL RESPIRATOR**

(30) Priority: 02.10.2020 JP 2020168124
(71) Applicant: Ishikita, Naoyuki, Kashiwazaki-shi, Niigata 945-8585 (JP)
(72) Inventor: Ishikita, Naoyuki, Kashiwazaki-shi, Niigata 945-8585 (JP)
(74) Representative: Schmid, Wolfgang
(86) International application number: PCT/JP2021/033852
(87) International publication number: WO 2022/070907

(57) **Abstract**

Provided is a new ventilator that does not require an electrical driving source and that can be used for cardiopulmonary resuscitation. A ventilator 1 includes a pipe 2 having a ventilation path 2e, a relief valve 3, and a spontaneous-breathing valve 4. The pipe 2 includes a main port 2b that allows a gas to be inhaled by a patient and a gas exhaled by the patient to pass therethrough, a relief valve port 2c that is in communication with the main port 2b through the ventilation path 2e, and a spontaneous-breathing valve port 2d that is in communication with the main port 2b through the ventilation path 2e. The relief valve 3 is mounted on the relief valve port 2c and configured to be opened in accordance with the pressure in the ventilation path 2e so as to be capable of allowing communication between the ventilation path 2e and the outside of the pipe 2 and releasing the pressure. The spontaneous-breathing valve 4 is mounted on the spontaneous-breathing valve port 2d, and the spontaneous-breathing valve 4 is configured to be opened in accordance with an intake pressure at which the patient spontaneously breathes and configured to be capable of allowing communication between the ventilation path 2e and the outside of the pipe 2 and enabling inhalation.

## Description

### Technical Field

The present disclosure relates to a ventilator.

### Background Art

The inventor of the present invention has proposed a relief valve that has a simple structure and that includes a valve body that automatically and properly performs opening and closing operations by using the pressure of a gas (PTL 1). This relief valve does not require an electrical drive source and can be used as, for example, an adjustable pressure limiting (APL) valve included in a ventilator or an inhalation anesthesia apparatus.

### Citation List

### Patent Literature

PTL 1: International Publication No. WO 2017/115866

### Summary of Invention

### Technical Problem

To the present inventor's knowledge, there is no ventilator device that has a simple structure without requiring an electrical drive source and that can be used as a ventilator like the above-mentioned relief valve. The present disclosure provides a new technology that can be used for cardiopulmonary resuscitation.

### Solution to Problem

An aspect of the present disclosure is a ventilator including a pipe having a ventilation path, a relief valve, and a spontaneous-breathing valve. The pipe includes a main port allowing a gas that is to be inhaled by a patient and a gas exhaled by the patient to pass through the main port, a relief valve port communicating with the main port through the ventilation path, and a spontaneous-breathing valve port communicating with the main port through the ventilation path. The relief valve is mounted on the relief valve port and configured to be opened in accordance with a pressure in the ventilation path in such a manner as to be capable of allowing communication between the ventilation path and an outside of the pipe and releasing the pressure. The spontaneous-breathing valve is mounted on the spontaneous-breathing valve port, and the spontaneous-breathing valve is configured to be opened in accordance with an intake pressure at which the patient spontaneously breathes and configured to be capable of allowing communication between the ventilation path and the outside of the pipe and enabling inhalation.

According to an aspect of the present disclosure, a ventilator having a simple structure can be fabricated.

### Brief Description of Drawings

[Fig. 1] Fig. 1 is a front view of a ventilator according to an embodiment.

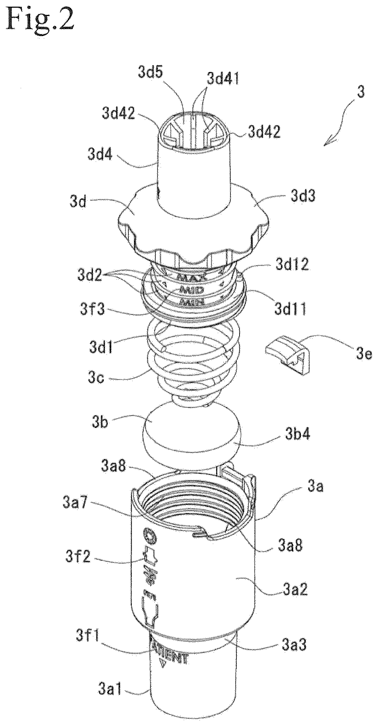

[Fig. 2] Fig. 2 is an exploded perspective view including the front surface, the right-side surface, and a flat surface of the ventilator illustrated in Fig. 1.

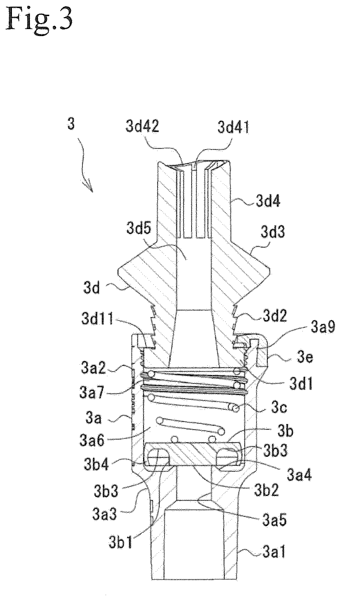

[Fig. 3] Fig. 3 is a sectional view of a relief valve taken along line III-III of Fig. 1.

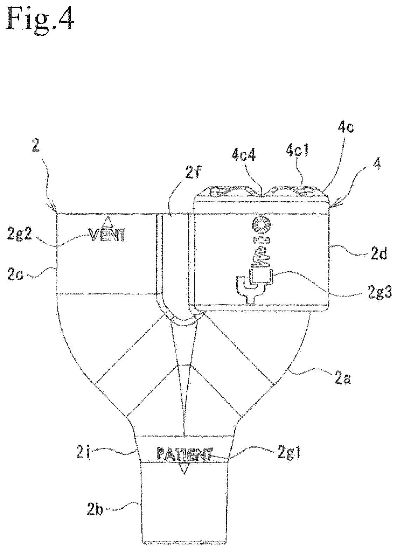

[Fig. 4] Fig. 4 is a front view of a pipe with a spontaneous-breathing valve for the ventilator illustrated in Fig. 1.

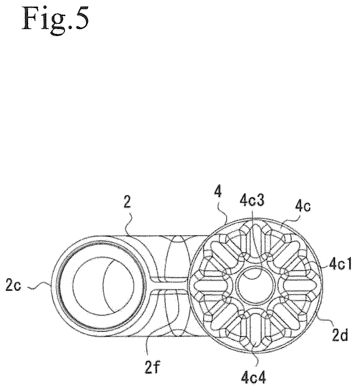

[Fig. 5] Fig. 5 is a plan view of the pipe with the spontaneous-breathing valve for the ventilator illustrated in Fig. 1.

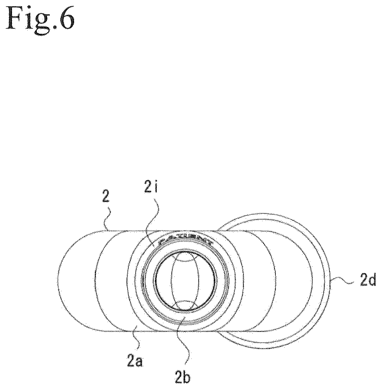

[Fig. 6] Fig. 6 is a bottom view of the pipe with the spontaneous-breathing valve for the ventilator illustrated in Fig. 1.

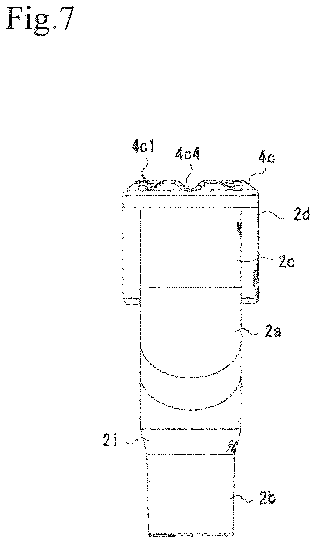

[Fig. 7] Fig. 7 is a right-hand side view of the pipe with the spontaneous-breathing valve for the ventilator illustrated in Fig. 1.

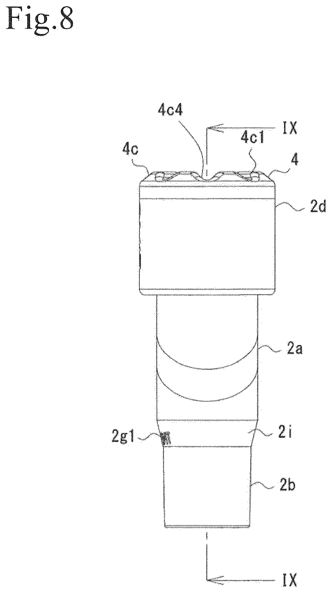

[Fig. 8] Fig. 8 is a left-hand side view of the pipe with the spontaneous-breathing valve for the ventilator illustrated in Fig. 1.

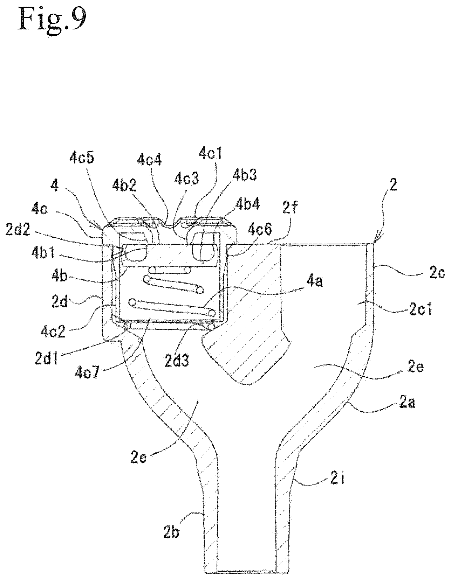

[Fig. 9] Fig. 9 is a sectional view taken along line IX-IX of Fig. 8.

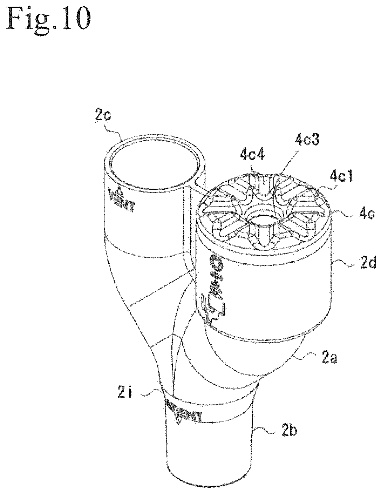

[Fig. 10] Fig. 10 is a perspective view including the front surface, the right-side surface, and a flat surface of the pipe with the spontaneous-breathing valve for the ventilator illustrated in Fig. 1.

### Description of Embodiments

An exemplary embodiment according to an aspect of the present disclosure will be described below with reference to the drawings. The embodiment, which will be described below, does not unreasonably limit the scope of the present invention described in the claims, and not all the configurations in the description of the embodiment are essential as the solutions of the present invention. In the following description, terms indicating directions such as "upper", "lower", "left", and "right" are used for convenience of description and are not intended to describe how to use a ventilator 1 or a mode of use of the ventilator 1. The terms "first", "second", ..., and "seventh" that are mentioned in the present specification and the claims are used as identification terms that distinguish different components in the invention and the embodiment and are not used for indicating either a specific order or superiority. Consequently, larger ordinal number terms such as "eighth" that are not mentioned in the original specification may sometimes be used.

The ventilator 1 includes a pipe 2, a relief valve 3, and a spontaneous-breathing valve 4. The ventilator 1 can be configured with a small number of components and a simple configuration.

### Description of Pipe 2

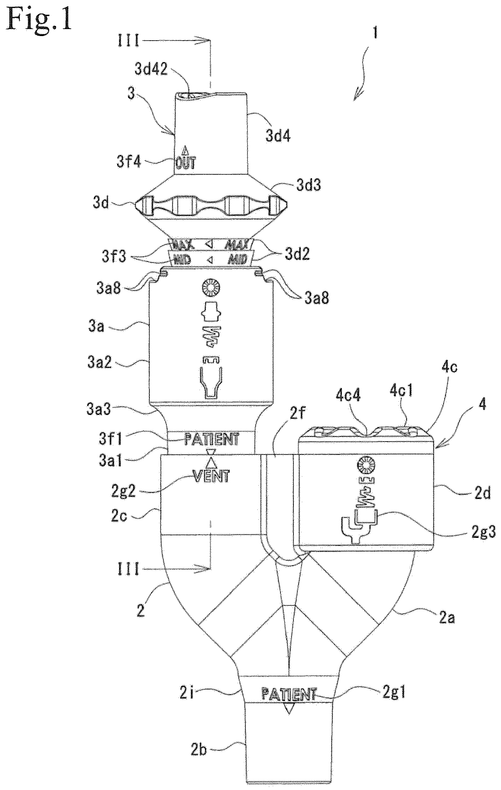

The pipe 2 is formed of a hollow pipe. The pipe 2 of the present embodiment is a resin-molded body (a 3D printed body) formed by using a 3D printer. Thus, the pipe 2 can be easily manufactured without using a metal mold. The entire pipe 2 is formed as a branch pipe. More specifically, the pipe 2 includes a main body portion 2a that is branched into a plurality of portions, a main port 2b, a relief valve port 2c, and a spontaneous-breathing valve port 2d. The main body portion 2a is branched so as to have a Y-shape, and as illustrated in Fig. 1, the main port 2b is attached to the lower end of the Y-shape. The relief valve port 2c is attached to the upper left end of the Y-shape. The spontaneous-breathing valve port 2d is attached to the upper right end of the Y-shape. A ventilation path 2e is formed inside the above-described pipe 2. The ventilation path 2e of the present embodiment has a Y-shape that is the same as the shape of the main body portion 2a.

Here, a support portion 2f is formed between the relief valve port 2c and the spontaneous-breathing valve port 2d that branch off from the main body portion 2a. The support portion 2f is provided so as to facilitate fixation to a build platform and so as to prevent the pipe 2 from warping when 3D printing is performed. In other words, the support portion 2f is provided, so that the pipe 2 can be shaped and formed without a raft or a support member, which are required when 3D printing is performed. In addition, the support portion 2f is formed as a reinforcement wall connecting the relief valve port 2c and the spontaneous-breathing valve port 2d to each other. A Y-shaped pipe generally bifurcates into two portions, which are an upper left pipe and an upper right pipe, and there is a space between the upper left pipe and the upper right pipe of the Y-shaped pipe. Thus, when the Y-shaped pipe is hit or dropped, the upper left pipe and the upper right pipe may sometimes be broken and damaged. In contrast, in the pipe 2 of the present embodiment, the support portion 2f can reinforce the relief valve port 2c and the spontaneous-breathing valve port 2d by connecting them integrally. The support portion 2f of the present embodiment has a thin-plate-like shape. More specifically, the support portion 2f is formed so as to have a thickness approximately equal to the thickness of the relief valve port 2c and approximately equal to the thickness of the spontaneous-breathing valve port 2d. Thus, an increase in the weight of the pipe 2 (the ventilator 1) can be suppressed even though the support portion 2f is provided, and the ventilator 1 can be configured to be lightweight.

The pipe 2 is provided with display portions 2g. More specifically, a first display portion 2g1 is provided at a position adjacent to the main port 2b. A second display portion 2g2 is provided at a position adjacent to the relief valve port 2c. A third display portion 2g3 is provided at a position adjacent to the spontaneous-breathing valve port 2d. The first display portion 2g1, the second display portion 2g2, and the third display portion 2g3 are each formed as a surface portion that has irregularities and that is included in a surface of the resin-molded body. Thus, these display portions will not disappear as if, for example, stickers come off when the ventilator 1 is used.

The first display portion 2g1 includes a character string "PATIENT" and an alignment symbol. The alignment symbol of the present embodiment is a triangle symbol. The first display portion 2g1 indicates that the main port 2b is a patient-side connection end of the ventilator 1. When the ventilator 1 is used, the main port 2b can be correctly connected to a patient-side breathing member by checking the first display portion 2g1.

The second display portion 2g2 includes a character string "VENT" and an alignment symbol. The alignment symbol of the present embodiment is a triangle symbol. The character string "VENT" of the second display portion 2g2 is an abbreviation for "VENTIRATOR" and indicates that the relief valve port 2c is a connection port to which the relief valve 3 is connected in the ventilator 1. The second display portion 2g2 includes the alignment symbol (the triangle symbol), so that it can be easily aligned with an alignment symbol (a triangle symbol) of the relief valve 3, which will be described later, and the relief valve 3 can be properly assembled to the relief valve port 2c.

The third display portion 2g3 includes assembly instruction symbols that indicate a procedure of assembling the spontaneous-breathing valve 4 to the spontaneous-breathing valve port 2d. The assembly instruction symbols are component symbols resembling the shapes of components. As illustrated in Fig. 1, the third display portion 2g3 includes component symbols of the pipe 2, a pressing spring 4a, an intake valve 4b, and a lid member 4c, and these component symbols are arranged in this order from the lower side. By displaying the plurality of component symbols in the assembly order as mentioned above, the components that are necessary for the spontaneous-breathing valve 4 can be confirmed, and the plurality of components of the spontaneous-breathing valve 4 can be assembled in a correct direction and a correct order.

The main port 2b, the relief valve port 2c, and the spontaneous-breathing valve port 2d each have a cylindrical shape. However, since the support portion 2f is formed between the relief valve port 2c and the spontaneous-breathing valve port 2d, it cannot be said that the relief valve port 2c and the spontaneous-breathing valve port 2d each have a completely cylindrical shape along its whole periphery. In this manner, the main port 2b, the relief valve port 2c, and the spontaneous-breathing valve port 2d have different inner diameters, different outer diameters, and different outer peripheral shapes from one another. Consequently, the patient-side breathing member that is to be connected to the main port 2b cannot be connected to either the relief valve port 2c or the spontaneous-breathing valve port 2d, and vice versa. This prevents incorrect assembly of the ventilator 1.

The main port 2b has a cylindrical shape. The main port 2b is formed so as to have an outer diameter smaller than that of an annular inclined portion 2i of the main body portion 2a that is provided with the first display portion 2g1. The main port 2b is inserted into the patient-side breathing member. In this case, an end of the patient-side breathing member inserted in the main port 2b can come into contact with the annular inclined portion 2i, and thus, the annular inclined portion 2i functions as a stopper that stops excessive insertion of the main port 2b into the patient-side breathing member. Therefore, the patient-side breathing member can be properly connected to the main port 2b.

Examples of the "patient-side breathing member", which is to be connected to the main port 2b, can include a connection port of a heat-and-moisture exchanger (HME) unit, a connection port of a corrugated tube connected to an HME unit, a connection port of a resuscitation mask to be worn on a patient, a connection port of a corrugated tube connected to a resuscitation mask, a nasal or oral endotracheal intubation tube, a tracheotomy tube, and a connection port of a supraglottic device (a laryngeal mask). The main port 2b can be easily connected to these patient-side breathing members by being inserted into the patient-side breathing members. In particular, even at treatment sites, such as emergency situations, where medical facilities and medical equipment are not available, the main port 2b can be easily connected to an endotracheal intubation tube or the like, and the ventilator 1 can be easily and quickly assembled and used.

The relief valve port 2c has a cylindrical shape and is connected to the support portion 2f at a point on the outer periphery thereof. More specifically, the support portion 2f is connected to the relief valve port 2c and extends so as to reach an open-side end portion of the relief valve port 2c. This can prevent, for example, the patient-side breathing member from being connected to the outer periphery of the relief valve port 2c by mistake. The inner peripheral surface of the relief valve port 2c serves as a relief-valve holding portion 2c1 into which a connection portion 3a1 of the relief valve 3, which will be described later, is press-fitted. Thus, the relief valve 3 can be easily attached to the pipe 2 by being inserted into the relief valve port 2c.

The spontaneous-breathing valve port 2d has a cylindrical shape. Similar to the above-described relief valve port 2c, the spontaneous-breathing valve port 2d is connected to the support portion 2f at a point on the outer periphery thereof. More specifically, the support portion 2f is connected to the spontaneous-breathing valve port 2d and extends so as to reach an open-side end portion of the spontaneous-breathing valve port 2d. This can prevent, for example, the patient-side breathing member from being connected to the outer periphery of the spontaneous-breathing valve port 2d by mistake. In addition, the inner diameter of the spontaneous-breathing valve port 2d is larger than the inner diameter of the relief valve port 2c. Thus, even if the connection portion 3a1 of the relief valve 3 is inserted into the spontaneous-breathing valve port 2d by mistake, it cannot be fixed onto the spontaneous-breathing valve port 2d because there will be a large gap between the connection portion 3a1 and the spontaneous-breathing valve port 2d. Therefore, the relief valve 3 cannot be attached to the spontaneous-breathing valve port 2d.

An accommodation portion 2d1 is formed inside the spontaneous-breathing valve port 2d. A fit-receiving portion 2d2 is formed on the upper end side of the accommodation portion 2d1 (an open end side of the spontaneous-breathing valve port 2d), and the lid member 4c of the spontaneous-breathing valve 4, which will be described later, is press-fitted and fixed into the fit-receiving portion 2d2. The fit-receiving portion 2d2 of the present embodiment is formed as a fitting groove. Thus, the lid member 4c of the spontaneous-breathing valve 4 can be easily fitted into the fit-receiving portion 2d2 by pushing the lid member 4c into the fit-receiving portion 2d2 and can be fixed to the spontaneous-breathing valve port 2d.

### Description of Relief Valve 3

The relief valve 3 includes a main body portion 3a, a pressure adjustment valve 3b, a pressure adjustment spring 3c, a pressure-adjustment operating unit 3d, and a stopper 3e. The relief valve 3 is configured to function as an adjustable pressure limiting (APL) valve. The main body portion 3a, the pressure adjustment valve 3b, the pressure adjustment spring 3c, the pressure-adjustment operating unit 3d, and the stopper 3e that are included in the relief valve 3 of the present embodiment are each a resin-molded body (a 3D printed body) formed by using a 3D printer. Thus, the relief valve 3 can be easily manufactured without using a metal mold.

The main body portion 3a has a cylindrical shape. The main body portion 3a includes the connection portion 3a1 and a valve casing 3a2. The connection portion 3a1 has a cylindrical shape and is inserted into the relief valve port 2c, so that the relief valve 3 is fixed onto the pipe 2. As a result, the relief valve 3 is formed as an integral structure with the pipe 2. On the other hand, the relief valve 3 can be detached from the pipe 2 by removing the connection portion 3a1 from the relief valve port 2c. In other words, the relief valve 3 is configured to be attachable and detachable to and from the pipe 2. The outer peripheral surface of the connection portion 3a1 has a fourth display portion 3f1 including a character string "PATIENT" and an alignment symbol. The alignment symbol of the present embodiment is a triangle symbol, and this triangle symbol and the triangle symbol that is included in the above-described second display portion 2g2 are aligned with each other, so that the relief valve 3 can be properly connected to the relief valve port 2c.

An increased-diameter portion 3a3, which is formed of a curved outer peripheral surface, is formed at the upper end of the connection portion 3a1. A valve seat 3a4 is formed on the inner peripheral surface of the increased-diameter portion 3a3, and the valve seat 3a4 has a valve hole 3a5 extending therethrough in an axial direction of the main body portion 3a. The valve casing 3a2 is located on the secondary side of the valve hole 3a5 in a ventilation direction that is a direction from the connection portion 3a1 toward the pressure-adjustment operating unit 3d, and a valve chamber 3a6 is formed inside the valve casing 3a2. The valve chamber 3a6 is formed as a columnar accommodation space, and the pressure adjustment valve 3b and the pressure adjustment spring 3c are accommodated in the valve chamber 3a6. A pressing-amount adjustment portion 3a7 is formed at the upper end of the valve chamber 3a6 (a secondary-side open end of the main body portion 3a). The pressing-amount adjustment portion 3a7 of the present embodiment is formed as a thread-receiving portion that engages with a threaded portion 3d1 of the pressure-adjustment operating unit 3d, which will be described later.

The outer peripheral surface of the main body portion 3a has a fifth display portion 3f2 that includes assembly instruction symbols indicating a procedure of assembling the relief valve 3 and that is a part of the surface shape of the relief valve 3. The fifth display portion 3f2 includes component symbols of the main body portion 3a, the pressure adjustment valve 3b, the pressure adjustment spring 3c, and the pressure-adjustment operating unit 3d. The plurality of component symbols are displayed in the assembling order as mentioned above, so that the components that are necessary for the relief valve 3 can be confirmed, and the plurality of components of the relief valve 3 can be assembled in a correct direction and a correct order.

A pressure indication portion 3a8 that indicates an operating pressure of the pressure adjustment valve 3b that is currently set is formed at the upper end of the main body portion 3a. The pressure indication portion 3a8 is formed as a recess by partially cutting out an upper-end opening edge of the main body portion 3a, and a set-pressure display unit 3d2 that is positioned within a range of the height of the recess and that will be described later, indicates the currently set operating pressure. In this manner, the shape of the recess enables the set-pressure display unit 3d2 to display the currently set operating pressure in an easy-to-understand manner.

A stopper attachment portion 3a9 is formed at the upper end of the main body portion 3a. The stopper attachment portion 3a9 is formed as a mounting base of the above-mentioned stopper 3e. The stopper 3e can be easily mounted on the stopper attachment portion 3a9 by being press-fitted into the stopper attachment portion 3a9, so that the pressure-adjustment operating unit 3d can be prevented from being separated from the main body portion 3a.

The pressure adjustment valve 3b has a disc-like shape. A protruding portion 3b1 is formed on a surface of the pressure adjustment valve 3b, the surface being located on the primary side in the ventilation direction. The valve hole 3a5 is opened and closed as a result of an end surface 3b2 of the protruding portion 3b1 moving into and out of contact with the valve seat 3a4. In other words, the end surface 3b2 is formed as a "first pressure-receiving surface", and the pressure adjustment valve 3b is operated so as to be opened by the air pressure exerted on the end surface 3b2. An annular pressure-receiving portion 3b3 is formed at the outer periphery of the protruding portion 3b1 so as to surround the protruding portion 3b1. The annular pressure-receiving portion 3b3 is formed as a "second pressure-receiving surface" that receives the pressure of the air that flows into the valve chamber 3a6 when the end surface 3b2 moves away from the valve seat 3a4. Thus, the inner surface of the annular pressure-receiving portion 3b3 is formed as a curved concave surface with no corners such that the air that flows into the annular pressure-receiving portion 3b3 smoothly flows without becoming stagnant. In addition, the annular pressure-receiving portion 3b3 receives the air pressure, so that the pressure adjustment valve 3b moved away from the valve hole 3a5 can be displaced so as to move farther away from the valve hole 3a5. An annular surrounding wall portion 3b4 is formed on the outside of the annular pressure-receiving portion 3b3. The inner peripheral surface of the annular surrounding wall portion 3b4 forms the annular pressure-receiving portion 3b3, and a ventilation gap is formed between the outer peripheral surface of the annular surrounding wall portion 3b4 and the inner peripheral surface of the valve casing 3a2, which forms the valve chamber 3a6. The air flows out through the ventilation gap to a surface of the pressure adjustment valve 3b, the surface being located on the secondary side.

The pressure adjustment spring 3c is formed of a compression-coil spring having a conical shape and is a member for setting the operating pressure of the pressure adjustment valve 3b (a valve-opening pressure). A smaller-diameter end portion of the pressure adjustment spring 3c that is located on the primary side is in contact with the secondary-side surface of the pressure adjustment valve 3b, and a larger-diameter end portion of the pressure adjustment spring 3c that is located on the secondary side is in contact with an end portion of the pressure-adjustment operating unit 3d.

The pressure-adjustment operating unit 3d has a function of adjusting a pressure for opening and closing the pressure adjustment valve 3b and a function of exhausting the air that flows into the valve chamber 3a6 to the outside. The threaded portion 3d1 is formed at an end of the pressure-adjustment operating unit 3d, the end being located on the primary side. An external thread is formed on the outer peripheral surface of the threaded portion 3d1 so as to engage with the pressing-amount adjustment portion 3a7 of the valve casing 3a2. By adjusting the degree of engagement between the threaded portion 3d1 and the pressing-amount adjustment portion 3a7, the magnitude of a pressure that is continuously applied to the pressure adjustment spring 3c and the operating pressures at which the pressure adjustment valve 3b is opened and closed can be set. In this manner, the operating pressure of the pressure adjustment valve 3b can be adjusted by a simple operation of rotating the pressure-adjustment operating unit 3d.

The threaded portion 3d1 has an engagement surface 3d11 that comes into contact with the stopper 3e so as to prevent the pressure-adjustment operating unit 3d from coming off from the main body portion 3a. In addition, a rotation restricting protrusion 3d12 is formed on the engagement surface 3d11. When the pressure-adjustment operating unit 3d is continuously rotated in a direction in which the pressure-adjustment operating unit 3d disengages from the main body portion 3a, the rotation restricting protrusion 3d12 comes into contact with the stopper 3e. Then, the pressure-adjustment operating unit 3d cannot be rotated any more. This state in which the degree of engagement is lowest is the state in which the pressure at which the pressure adjustment valve 3b operates is set to the minimum value (the state in which the lowest operating pressure (the lowest valve-opening pressure of the pressure adjustment valve 3b) is set), and more specifically, the minimum value is 5 cmH₂O that is required for a standard (e.g., IS010651-4:2002) required for a manual pulmonary resuscitator.

The operating pressure may be set by visually observing the set-pressure display unit 3d2 formed above the threaded portion 3d1. The set-pressure display unit 3d2 includes a plurality of sixth display portions 3f3. The sixth display portions 3f3 are formed by arranging a plurality of annular surfaces, each of which has a frustoconical shape, in multiple stages such that they are continuous with one another in an axial direction of the relief valve 3. The frustoconical annular surfaces each have one of character strings "MIN", "MID", and "MAX" each of which is formed as a part of the surface shape of the resin-molded body (each of which is formed as a recessed portion having the shapes of characters) in ascending order of the degree of engagement and the operating pressure.

A handle 3d3 is formed above the set-pressure display unit 3d2. The handle 3d3 has a disc-like shape greatly projecting outward so as to be easy to grip, so that a rotation operation of the handle 3d3 can be easily performed.

An exhaust pipe 3d4 is formed above the handle 3d3. The exhaust pipe 3d4 has a cylindrical shape. A plurality of inner walls 3d41 are formed on the inner peripheral surface of the exhaust pipe 3d4 in such a manner as to be radially arranged with respect to the central axis of the exhaust pipe 3d4. The inner walls 3d41 are formed in this manner, so that even a small-diameter medical tubular member, such as a tracheal cannula or endotracheal intubation tube, cannot be physically connected to the inside of the exhaust pipe 3d4. In addition, the outer diameter of the exhaust pipe 3d4 is set to be small in such a manner that the exhaust pipe 3d4 can be inserted into and connected to only a specific corrugated pipe (whose inner diameter is 18 mm) in order to prevent an improper connection.

The exhaust pipe 3d4 has degassing recesses 3d42 each of which is formed by partially cutting out an opening edge of the exhaust pipe 3d4 into an arc shape in a circumferential direction. In the case where the opening edge of the exhaust pipe 3d4 has the shape of a linear end surface with no irregularities, the exhaust pipe 3d4 is likely to be closed by a flat object. However, forming the degassing recesses 3d42 can make the exhaust pipe 3d4 less likely to be closed by a flat object, so that the relief valve 3 can properly operate.

The exhaust pipe 3d4 has a seventh display portion 3f4 that includes a character string "OUT" and a symbol indicating an exhaust direction and that is formed as a part of the surface shape of the resin-molded body. Accordingly, it can be easily understood that the exhaust pipe 3d4 is a portion through which the air is exhausted, that is, a portion to which attention should be paid and that should not be closed in order to allow the relief valve 3 to perform a proper pneumatic operation.

The pressure-adjustment operating unit 3d has an exhaust path 3d5 that is formed in such a manner as to extend therethrough along the central axis thereof. The exhaust path 3d5 is formed as a ventilation path that allows communication between the valve chamber 3a6 of the main body portion 3a and the outside of the relief valve 3.

### Description of Spontaneous-Breathing Valve 4

The spontaneous-breathing valve 4 is opened by an intake pressure at which a patient spontaneously breathes and functions so as to take the air into the ventilation path 2e of the pipe 2. The spontaneous-breathing valve 4 includes the pressing spring 4a, the intake valve 4b, and the lid member 4c. The spontaneous-breathing valve 4 is formed by assembling these components to the spontaneous-breathing valve port 2d. The components of the spontaneous-breathing valve 4 are each a resin-molded body (a 3D printed body) formed by using a 3D printer. Thus, the spontaneous-breathing valve 4 can be easily manufactured without using a metal mold.

The pressing spring 4a is formed of a compression-coil spring having a conical shape and is a member for setting the operating pressure of the intake valve 4b (a valve-opening pressure). A larger-diameter end portion of the pressing spring 4a, which is the primary-side end portion of the pressing spring 4a, is located on a support surface 2d3 of the accommodation portion 2d1 of the spontaneous-breathing valve port 2d. The support surface 2d3 is formed as a surface having a conical shape in such a manner that the pressing spring 4a is centered when it is incorporated into the accommodation portion 2d1. A smaller-diameter end portion of the pressing spring 4a, which is the secondary-side end portion of the pressing spring 4a, is in contact with the primary-side surface of the intake valve 4b and continuously presses the intake valve 4b.

The shape of the intake valve 4b is the same as that of the pressure adjustment valve 3b of the relief valve 3, which has been mentioned above. In other words, the intake valve 4b has a protruding portion 4b1, an end surface 4b2, an annular pressure-receiving surface 4b3, and an annular surrounding wall portion 4b4, and their functions are the same as those of the pressure adjustment valve 3b. Therefore, repeated descriptions will be omitted.

The lid member 4c includes a lid portion 4c1 and a mounting portion 4c2. The lid portion 4c1 has a disc-like shape, and an intake hole 4c3 is formed at the center of the lid portion 4c1 so as to extend through the lid portion 4c1. A plurality of ventilation grooves 4c4 are formed in the outer surface of the lid portion 4c1 so as to radially extend from the intake hole 4c3. Even if a flat object comes into contact with the outer surface of the lid portion 4c1, the ventilation grooves 4c4 serve as ventilation paths, and thus, the intake hole 4c3 will not be completely closed, so that the spontaneous-breathing valve 4 can properly operate. Therefore, the safety of the ventilator 1 can be improved. The inner surface of the lid portion 4c1 that is located on the side opposite to the side on which the ventilation grooves 4c4 has an intake-hole valve seat 4c5 that is formed so as to surround the intake hole 4c3. The intake valve 4b that receives the pressing force of the pressing spring 4a is in contact with the intake-hole valve seat 4c5. Thus, the intake valve 4b closes the intake-hole valve seat 4c5 until the intake pressure (the valve-opening pressure) at which a patient spontaneously breathes reaches a predetermined intake pressure (a predetermined valve-opening pressure). When the intake pressure exceeds a predetermined set value, the intake valve 4b becomes displaced against the pressing force of the pressing spring 4a, and the intake-hole valve seat 4c5 is opened.

The mounting portion 4c2 is formed as a portion extending from an outer peripheral edge of the inner surface of the lid portion 4c1 in such a manner as to have a cylindrical shape. The outer peripheral surface of the mounting portion 4c2 that is adjacent to the inner surface of the lid portion 4c1 has a fitting portion 4c6. The fitting portion 4c6 is pressed and fitted into the fit-receiving portion 2d2 of the spontaneous-breathing valve port 2d as mentioned above. As a result, the lid member 4c can be easily fixed onto the spontaneous-breathing valve port 2d.

The inner diameter of the mounting portion 4c2 is slightly larger than the outer diameter of the intake valve 4b. More specifically, the inner diameter of the mounting portion 4c2 is equal to the inner diameter of the main body portion 3a of the relief valve 3. In other words, similar to the pressure adjustment valve 3b of the relief valve 3, the intake valve 4b forms a ventilation gap between the intake valve 4b and the inner-diameter surface of the mounting portion 4c2. As a result, the intake valve 4b can be displaced in a stable position along a direction in which the central axis of the spontaneous-breathing valve 4 extends while the inner-diameter surface of the mounting portion 4c2 serves as a guide surface. Therefore, in the spontaneous-breathing valve 4, an inner space that is surrounded by the mounting portion 4c2 forms a valve chamber 4c7 in which the intake valve 4b operates.

### Method of Manufacturing Ventilator 1

In order to construct the ventilator 1, the components of the pipe 2, the components of the relief valve 3, and the components of the spontaneous-breathing valve 4 are formed by using a 3D printer. Then, the relief valve 3 is assembled in the order indicated by the fifth display portion 3f2. The spontaneous-breathing valve 4 is assembled to the spontaneous-breathing valve port 2d of the pipe 2 in the order indicated by the third display portion 2g3, which is one of the components of the spontaneous-breathing valve 4. As a result, the pipe 2 having a structure in which the spontaneous-breathing valve 4 is integrated therewith is formed. Lastly, the connection portion 3a1 of the relief valve 3 is connected to the relief valve port 2c of the pipe 2. In the manner described above, the ventilator 1 can be manufactured by simple assembly using only a 3D printer and without using a special assembly tool.

### Implementation Examples of Medical Device using Ventilator 1

As mentioned above, various "patient-side breathing members" can be connected to the main port 2b of the ventilator 1. Here, as an example, the main port 2b can be connected to a connection port of an HME unit, which is not illustrated. The HME unit has an input port, and a pipe for supplying air or a mixed gas of oxygen and air can be connected to the input port, or an air or oxygen cylinder, an air compressor, or the like can be connected to the input port via an oxygen tube. According to the ventilator 1 including such an HME unit, the ventilator 1 can be fabricated with a simple configuration including the HME unit.

A corrugated tube can be connected to the exhaust pipe 3d4, and for example, a purification filter for purifying an anesthetic gas contained in an exhausted gas (a gas exhaled by a patient) can be connected to the corrugated tube.

As described above, the ventilator 1 can be used for various purposes depending on the equipment that is combined with the ventilator 1, and some uses of the ventilator 1 will be described below as examples. The following implementation examples may suitably have a configuration including a corrugated tube or a breathing tube. In addition, for example, in each of the following implementation examples, a configuration may be employed in which a ventilation-volume measurement device such as a spirometer is provided between a patient-side breathing member, such as a resuscitation mask or an endotracheal intubation tube, and the main port 2b of the ventilator 1 such that a tidal volume (a respiratory volume) can be monitored.

### [1] Manual Pulmonary Resuscitator

As a first implementation example, the ventilator 1 includes at least the "patient-side breathing member", such as a resuscitation mask or an endotracheal intubation tube, that is worn on a patient and connected to the main port 2b of the ventilator 1 and a "manual gas supply device", such as a resuscitation bag or a foot pump, that is connected to an input port of, for example, the HME unit of the ventilator 1, so that the ventilator 1 can be implemented as a "manual pulmonary resuscitator". According this implementation example, a general manual pulmonary resuscitator can have a function of serving as a ventilator. Regarding a method of supplying the air, which is used as power, the air can be supplied without expert knowledge, and since the relief valve 14 can automatically adjust the pressure, the ventilator 1 can be safely used also by manually as long as the air is continuously supplied by squeezing a resuscitation bag or stepping on a foot pump. In addition, it will be convenient if a foot pump is used because ventilation can be performed by using a foot while both hands are free, so that a patient can be treated at the same time. This manual pulmonary resuscitator can further include a gas supply source that supplies oxygen gas or a mixed gas and that is connected to a manual gas supply device such as a resuscitation bag.

### [2] Ventilator Unit

As a second implementation example, the ventilator 1 includes at least the "patient-side breathing member", such as a mask or an intubation tube, that is worn on a patient and connected to the main port 2b of the ventilator 1 and a "gas supply source" that is connected to the input port of the ventilator 1 and that supplies, for example, a mixed gas of oxygen and air as a "gas", so that the ventilator 1 can be implemented as a pneumatically-operated "ventilator unit". According to this implementation example, artificial respiration can be performed even in a situation in which a ventilator equipped with an electrical driving source cannot be used, examples of the situation including during an earthquake, in case of power outage, and when all ventilators are in use and there is no available ventilator.

### [3] Inhalation anesthesia apparatus

As a third implementation example, the ventilator 1 includes at least the "patient-side breathing member", such as a mask, that is worn on a patient and connected to the main port 2b of the ventilator 1, an "anesthetic-gas supply source" that is connected to the input port of the ventilator 1 and that supplies an anesthetic gas as a "gas", and an "anesthetic-gas purification filter" that is connected to a corrugated tube connected to the exhaust pipe 3d4, so that the ventilator 1 can be implemented as an "inhalation anesthesia apparatus". According to the present implementation example, the inhalation anesthesia apparatus can be fabricated with a simple device structure.

### Advantageous Effects of Ventilator 1

The following description focuses on the configuration of a main embodiment, and advantageous effects corresponding to the configuration will be described.

The ventilator 1 includes the pipe 2 having the ventilation path 2e, the relief valve 3, and the spontaneous-breathing valve 4. The pipe 2 includes the main port 2b that allows a gas to be inhaled by a patient and a gas exhaled by the patient to pass therethrough, the relief valve port 2c that is in communication with the main port 2b through the ventilation path 2e, and the spontaneous-breathing valve port 2d that is in communication with the main port 2b through the ventilation path 2e. The relief valve 3 is mounted on the relief valve port 2c and configured to be opened in accordance with the pressure in the ventilation path 2e so as to be capable of allowing communication between the ventilation path 2e and the outside of the pipe 2 and releasing the pressure. The spontaneous-breathing valve 4 is mounted on the spontaneous-breathing valve port 2d and configured to be opened in accordance with the intake pressure, at which the patient spontaneously breathes, in such a manner as to be capable of allowing communication between the ventilation path 2e and the outside of the pipe 2 and enabling inhalation. As described above, the ventilator 1 does not require an electrical driving source, and a new device for a pulmonary resuscitator that functions as the ventilator 1 with a simple structure can be fabricated.

The main port 2b of the ventilator 1 is connected to, for example, an HME unit which is a "patient-side breathing member". In addition, the primary side of the HME unit is connected to another "patient-side breathing member" such as an endotracheal intubation tube that is placed in a patient. A mixed gas of oxygen and air or the like is supplied to the patient. In such a usage state, when the airway pressure of the patient exceeds a predetermined threshold, that is, the set operating pressure (the set valve-opening pressure) of the pressure adjustment valve 3b of the relief valve 3, the pressure adjustment valve 3b is opened, and the air is exhausted to the outside through the exhaust pipe 3d4. As a result, an excessive increase in the airway pressure of the patient can be suppressed.

The ventilator 1 can be configured as a single-use "disposable ventilator". This configuration can help to prevent infectious diseases from occurring due to the use of the same ventilator 1 for a plurality of patients.

All the components of the ventilator 1 of the above-described embodiment are formed of resin-molded bodies formed by 3D printing. It is not necessary to prepare large equipment such as a molding machine to perform metal molding, and the ventilator 1 can be manufactured by using a 3D printer. Thus, the ventilator 1 can be manufactured even in a remote area, an isolated island, an airplane, a spacecraft, a space station, or the like where it is difficult to install manufacturing equipment such as a resin molding machine. By forming the ventilator 1 out of a 3D printed body, it is possible to manufacture only a necessary number of ventilators 1 on demand, which is economical. In addition, by forming the ventilator 1 out of a 3D printed body, it can be easily enlarged and reduced in size, and thus, the ventilator 1 can be customized so as to fit for animals of different body sizes. Furthermore, the ventilator 1 can be configured as a "disposable ventilator" that does not need to be separated into metal members and resin members for disposal.

All the components of the ventilator 1 can be formed of resin-molded bodies, and an MRI room where metal objects cannot be taken into can be used while the ventilator 1 is worn. In addition, a reduction in the manufacturing costs can also be achieved.

The spontaneous-breathing valve 4 includes the intake valve 4b that operates in accordance with the intake pressure, and the spontaneous-breathing valve port 2d has the valve chamber 4c7 in which the intake valve 4b is accommodated in such a manner as to be openable and closable. According to this configuration, the spontaneous-breathing valve 4 can be integrated with the spontaneous-breathing valve port 2d of the pipe 2. Thus, even if a patient who is unconscious and is under artificial respiration using the ventilator 1 suddenly wakes up and takes a spontaneous deep breath, the spontaneous-breathing valve 4 opens and can help the patient breathe. In this case, the spontaneous-breathing valve 4, the ventilation path 2e of the main body portion 2a of the pipe 2, and the main port 2b form an inspiratory path. Therefore, the safety of the ventilator 1 can be further improved.

The spontaneous-breathing valve 4 includes the lid member 4c and the intake valve 4b. The lid member 4c has the mounting portion 4c2 that is to be mounted on the spontaneous-breathing valve port 2d and the intake hole 4c3 that allows communication between the inside of the spontaneous-breathing valve port 2d and the outside of the pipe 2. The intake valve 4b is operated by the intake pressure in such a manner as to open and close the intake hole 4c3. According to this configuration, the spontaneous-breathing valve 4 can be integrated with the spontaneous-breathing valve port 2d of the pipe 2. Therefore, the safety of the ventilator 1 can be further improved.

The spontaneous-breathing valve 4 includes the pressing spring 4a that presses the intake valve 4b against the intake hole 4c3 so as to close the intake hole 4c3 and that elastically deforms such that the intake valve 4b is displaced in accordance with the predetermined intake pressure. Consequently, the spontaneous-breathing valve 4 does not require an electrical driving source and can be pneumatically operated.

The pipe 2 can be formed of a branch pipe in which the ventilation path 2e includes a plurality of branch paths. According to this configuration, the configuration of the pipe 2 can be simplified, and the pipe 2 can be easily manufactured by 3D printing.

The relief valve 3 includes the pressure adjustment valve 3b that is openable and closable in accordance with the pressure in the ventilation path 2e and the main body portion 3a in which the pressure adjustment valve 3b is accommodated, and the pressure adjustment valve 3b and the main body portion 3a can each be formed of a 3D printed body made of a resin. According to this configuration, the relief valve 3 can be easily manufactured by using a 3D printer.

The spontaneous-breathing valve 4 includes the intake valve 4b that operates in accordance with the intake pressure and the lid member 4c that is mounted on the spontaneous-breathing valve port 2d in such a manner that the intake valve 4b is accommodated in the spontaneous-breathing valve port 2d, and the intake valve 4b and the lid member 4c can each be formed of a 3D printed body made of a resin. According to this configuration, the spontaneous-breathing valve 4 can be easily manufactured by using a 3D printer.

### Modifications of Embodiment

In the above-described embodiment, although all the components of the ventilator 1 are formed of 3D printed bodies as an example, each of the components may be a resin-molded body formed by using a metal mold.

In the above-described embodiment, although each of the components of the ventilator 1 is made of a resin, a subset or all of the components may be made of a metal. In the case where a subset or all of the components is made of a metal, regardless of a difference in a basic manufacturing method, the subset or all of the components may be any member, such as a cut body, a casted body, or a 3D printed body formed by using a metal 3D printer, as long as it is made of a metal material. As a result, the durability of the ventilator 1 and its components can be improved. For example, although a case has been described in which the pressure adjustment spring 3c and the pressing spring 4a are resin-molded bodies, they may be metal springs.

In the above-described embodiment, although the pipe 2 includes the support portion 2f as an example, the support portion 2f may be removed such that a space is formed between the relief valve port 2c and the spontaneous-breathing valve port 2d.

In the above-described embodiment, although the pipe 2 (the main body portion 2a) has a Y-shape as an example, it may have a T-shape. Instead of a shape having three open ends, a shape having four or more additional open ends may be employed, and at least one of the open ends that is not used may be closed by a removable lid.

In the above-described embodiment, as an example, although the pipe 2 does not have an input port for introducing a gas into the ventilation path 2e, the pipe 2 may have an input port. A pipe for supplying air or a mixed gas of oxygen and air can be connected to the input port, or an air or oxygen cylinder, an air compressor, or the like can be connected to the input port via an oxygen tube.

In the above-described embodiment, although the first display portion 2g1 to the seventh display portion 3f4 are each provided as a part of the surface shape of the corresponding resin-molded body as an example, they may be formed of laser-engraved portions or may be formed by attaching printed stickers.

Note that, although the embodiment has been described in detail above, those skilled in the art will easily understand that many modifications can be made without substantially departing from the configurations and the advantageous effects of the present invention. Therefore, all such modifications are included in the scope of the present invention.

For example, in the specification or the drawings, a term that is mentioned at least once together with a different term that has a wider meaning or a similar meaning can be replaced with the different term anywhere in the specification or the drawings. In addition, the configurations and the operations of the ventilator 1, the ventilator unit, the manual pulmonary resuscitator, and the inhalation anesthesia apparatus are not limited to those in the above-described embodiment of the present invention, and various modifications can be made.

## Claims

1. A ventilator comprising:
a pipe having a ventilation path;
a relief valve; and
a spontaneous-breathing valve;
wherein the pipe includes
a main port allowing a gas that is to be inhaled by a patient and a gas exhaled by the patient to pass through the main port,
a relief valve port communicating with the main port through the ventilation path, and
a spontaneous-breathing valve port communicating with the main port through the ventilation path,
wherein the relief valve is mounted on the relief valve port and configured to be opened in accordance with a pressure in the ventilation path in such a manner as to be capable of allowing communication between the ventilation path and an outside of the pipe and releasing the pressure, and
wherein the spontaneous-breathing valve is mounted on the spontaneous-breathing valve port, and the spontaneous-breathing valve is configured to be opened in accordance with an intake pressure at which the patient spontaneously breathes and configured to be capable of allowing communication between the ventilation path and the outside of the pipe and enabling inhalation.

2. The ventilator according to Claim 1,
wherein the spontaneous-breathing valve includes an intake valve that operates in accordance with the intake pressure, and
wherein the spontaneous-breathing valve port has a valve chamber in which the intake valve is accommodated in such a manner as to be openable and closable.

3. The ventilator according to Claim 1,
wherein the spontaneous-breathing valve includes
a lid member having a mounting portion that is to be mounted on the spontaneous-breathing valve port and an intake hole that allows communication between an inside of the spontaneous-breathing valve port and the outside of the pipe, and
an intake valve that is operated by the intake pressure in such a manner as to open and close the intake hole.

4. The ventilator according to Claim 3,
wherein the spontaneous-breathing valve includes
a pressing member that presses the intake valve against the intake hole in such a manner as to close the intake hole and that elastically deforms such that the intake valve is displaced in accordance with the intake pressure, which is predetermined.

5. The ventilator according to any one of Claims 1 to 4,
wherein the pipe is a branch pipe in which the ventilation path includes a plurality of branch paths.

6. The ventilator according to any one of Claims 1 to 5,
wherein the pipe has an input port through which a gas is introduced into the ventilation path.

7. The ventilator according to any one of Claims 1 to 6,
wherein the pipe is a 3D printed body made of a resin.

8. The ventilator according to Claim 1,
wherein the relief valve includes a pressure adjustment valve that is openable and closable in accordance with the pressure in the ventilation path and a main body portion in which the pressure adjustment valve is accommodated, and
wherein the pressure adjustment valve and the main body portion are each a 3D printed body made of a resin.

9. The ventilator according to Claim 1,
wherein the spontaneous-breathing valve includes an intake valve that operates in accordance with the intake pressure and a lid member that is mounted on the spontaneous-breathing valve port in such a manner that the intake valve is accommodated in the spontaneous-breathing valve port, and
wherein the intake valve and the lid member are each a 3D printed body made of a resin.

10. The ventilator according to any one of Claims 1 to 9, further comprising:
a heat-and-moisture exchanger unit communicating with the main port.

11. An ventilator unit comprising:
the ventilator according to any one of Claims 1 to 10; and
a patient-side breathing member having one end connected to the main port of the ventilator and another end connected to a patient.

12. A manual pulmonary resuscitator comprising:
the ventilator according to any one of Claims 1 to 10.

13. An inhalation anesthesia apparatus comprising:
the ventilator according to any one of Claims 1 to 10.
